# EUROPEAN PATENT APPLICATION

(11) **EP 3 811 924 A1**
(43) Date of publication of application: **28.04.2021**
(21) Application number: 19810088.5
(22) Date of filing: 14.05.2019
(51) Int. Cl.: A61K 8/81, A61K 8/04, A61K 8/19, A61K 8/25, A61K 8/27, A61K 8/29, A61K 8/90, A61Q 17/04

(54) **BLOCK COPOLYMER-CONTAINING INORGANIC PARTICLE DISPERSION FOR COSMETICS**

(30) Priority: 29.05.2018 JP 2018102631
(71) Applicant: Shiseido Company, Ltd., Chuo-ku Tokyo 104-0061 (JP)
(72) Inventor: MIYAZAWA, Kazuyuki, Tokyo 104-0061 (JP); ITO, Yuji, Tokyo 104-0061 (JP)
(74) Representative: Santarelli
(86) International application number: PCT/JP2019/019177
(87) International publication number: WO 2019/230378

(57) **Abstract**

The invention provides an inorganic particle dispersion comprising a novel block copolymer capable of improving dispersibility of inorganic particles, particularly an inorganic particle dispersion for a cosmetic.

The inorganic particle dispersion, particularly the inorganic particle dispersion for a cosmetic of the present invention comprises a dispersion medium, an inorganic particle dispersed in the dispersion medium, and a block copolymer comprising a hydrophobic segment and a hydrophilic segment, wherein the hydrophobic segment comprises a monomer unit composed of at least one monomer selected from the following Formula 1 and Formula 2, wherein at least a portion of the hydrophilic segment is adsorbed on the inorganic particle, and the hydrophobic segment is oriented outwardly relative to the inorganic particle: 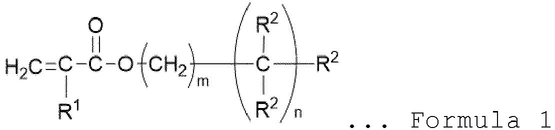 where R¹ is hydrogen or a methyl group, R² is hydrogen or fluorine, m is an integer of 0 to 6, and n is an integer of 1 to 15, 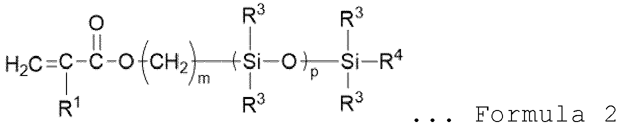 where R¹ is hydrogen or a methyl group, R³ and R⁴ are each independently an alkyl group having 1 to 6 carbon atoms, m is an integer of 1 to 6, and p is an integer of 5 to 70.

## Description

### FIELD

The present invention relates to an inorganic particle dispersion comprising a novel block copolymer, and particularly, to an inorganic particle dispersion for cosmetics.

### BACKGROUND

Recently, various surface modification techniques using polymer materials and the like have been studied for the purpose of imparting antifouling property, antifogging property, dispersibility of inorganic particles and the like.

Patent Literature 1 discloses an ink for ink jet recording containing water, a water-soluble compound, a pigment, and a (meth) acrylic ester-based random copolymer having an acid value of 100mgKOH/g or more and 160mgKOH/g or less in which the pigment is dispersed.

Patent Literature 2 discloses a block polymer which includes at least a hydrophobic segment and a hydrophilic segment, wherein the hydrophilic segment includes at least a monomer unit of a cationic monomer and an anionic monomer, and which can impart antifogging property, antistatic property, and the like to various members.

Patent Literature 3 discloses a pigment dispersion containing a pigment, a liquid medium and a polymer dispersant, wherein the polymer dispersant is a block polymer represented by A-B or A-B-C, the A block and the C block are polymer blocks composed of an ethylenically unsaturated monomer without amino and hydroxyl groups, and the B block is a polymer block in which either one of the amino compound and the compound having a hydroxyl group is bonded to a polymer block composed of a monomer having a glycidyl group or an isocyanate group via the glycidyl group or the isocyanate group.

### [CITATION LIST]

### [PATENT LITERATURE]

[PTL 1] Japanese Unexamined Patent Publication (Kokai) No. 2015-052058
[PTL 2] Japanese Unexamined Patent Publication (Kokai) No. 2017-179112
[PTL 3] International Publication No. WO 2010/016523

### SUMMARY

### [TECHNICAL PROBLEM]

For example, in the field of cosmetics for sunscreen, inorganic particles such as titanium oxide and the like are used for scattering ultraviolet rays. Since inorganic particles have a hydrophilic surface and tend to aggregate and precipitate when blended in an oil phase, for example, a surface treatment for improving dispersibility has been generally applied even for inorganic particles in such fields. However, in the conventional surface treatment, aggregation and precipitation of inorganic particles cannot be sufficiently prevented in some cases.

Accordingly, it is a subject of the present invention to provide an inorganic particle dispersion comprising a novel block copolymer capable of improving dispersibility and the like of inorganic particles, particularly an inorganic particle dispersion for cosmetics.

### [SOLUTION TO PROBLEM]

### <Aspect 1>

An inorganic particle dispersion for a cosmetic comprising a dispersion medium, an inorganic particle dispersed in the dispersion medium, and a block copolymer comprising a hydrophobic segment and a hydrophilic segment,
wherein the hydrophobic segment comprises a monomer unit composed of at least one monomer selected from the following Formula 1 and Formula 2,
wherein at least a portion of the hydrophilic segment is adsorbed on the inorganic particle, and the hydrophobic segment is oriented outwardly relative to the inorganic particle: 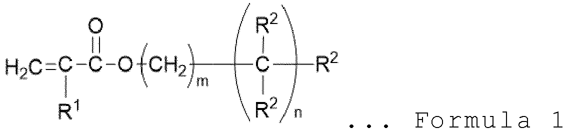
where R¹ is hydrogen or a methyl group,
R² is hydrogen or fluorine,
m is an integer of 0 to 6, and
n is an integer of 1 to 15,

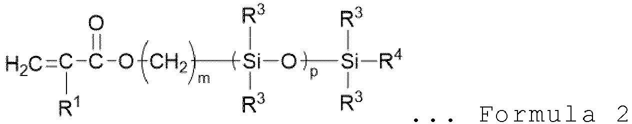

where R¹ is hydrogen or a methyl group,
R³ and R⁴ are each independently an alkyl group having 1 to 6 carbon atoms,
m is an integer of 1 to 6, and
p is an integer of 5 to 70.

### <Aspect 2>

The dispersion according to aspect 1, wherein the hydrophilic segment comprises a monomer unit composed of at least one monomer selected from Formula 3 to Formula 9 below. 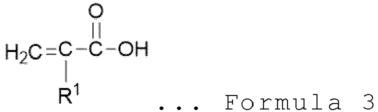 where R¹ is hydrogen or a methyl group, 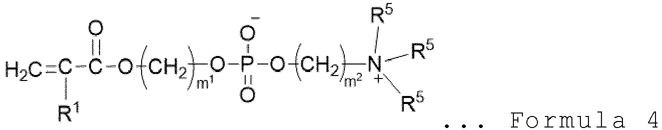
where R¹ is hydrogen or a methyl group,
m¹ and m² are each independently integers of 1 to 6 and
R⁵ is each independently an alkyl group having 1 to 6 carbon atoms,

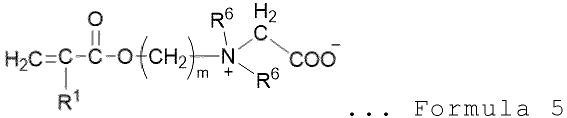

where R¹ is hydrogen or a methyl group,
m is an integer of 1 to 6, and
R⁶ is each independently an alkyl group having 1 to 6 carbon atoms,

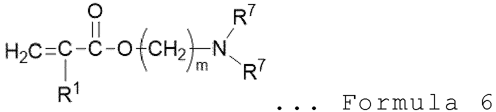

where R¹ is hydrogen or a methyl group,
m is an integer of 1 to 6, and
R⁷ is each independently an alkyl group having 1 to 6 carbon atoms,

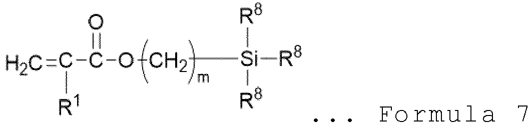

where R¹ is hydrogen or a methyl group,
m is an integer of 1 to 6, and
R⁸ is each independently a functional group that hydrolyzes and cross-links,

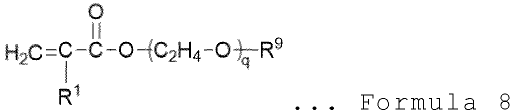

where R¹ is hydrogen or a methyl group,
q is an integer of 1 to 20, and
R⁹ is an alkyl group having 1 to 6 carbon atoms,

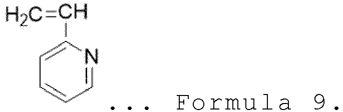

### <Aspect 3>

The dispersion according to aspect 1 or 2, wherein in the block copolymer, the proportion of the hydrophobic segment is from 50 to 99 mol% and the proportion of the hydrophilic segment is from 1 to 50 mol%.

### <Aspect 4>

The dispersion according to any one of aspects 1 to 3, wherein the proportion of the inorganic particle and the block copolymer is from 100:5 to 100:40 by mass.

### <Aspect 5>

The dispersion according to any one of aspects 1 to 4, wherein the inorganic particle is at least one selected from a titanium oxide particle, a zinc oxide particle, a cerium oxide particle, an iron oxide particle, and a mica particle.

### <Aspect 6>

An inorganic particle powder, wherein the dispersion according to any one of aspects 1 to 5 is dried.

### (Aspect 7>

A sunscreen cosmetic comprising the dispersion according to any one of aspects 1 to 5 or the inorganic particle powder according to aspect 6.

### <Aspect 8>

The cosmetic according to aspect 7, wherein the SPF value is 15 or more and the viscosity is 50000Pa·s or less.

### <Aspect 9>

The cosmetic according to aspect 7 or 8, wherein the inorganic particle is contained in an amount of 5% by mass or more and 50% by mass or less based on the total amount of the cosmetic.

### [ADVANTAGEOUS EFFECTS OF INVENTION]

According to the present invention, it is possible to provide an inorganic particle dispersion containing a novel block copolymer capable of improving dispersibility and the like of inorganic particles, particularly an inorganic particle dispersion for cosmetics.

### BRIEF DESCRIPTION OF DRAWINGS

FIG. 1(a) is a schematic diagram of an inorganic particle surface-treated with a block copolymer, FIG. 1(b) is a schematic diagram of an inorganic particle surface-treated with a random copolymer, and FIG. 1(c) is a schematic diagram of an inorganic particle surface-treated with a surface treatment agent of a low-molecular type.
FIG. 2 is a schematic diagram of an inorganic particle surface-treated with the block copolymer of one embodiment of the present invention.

### DESCRIPTION OF EMBODIMENTS

Hereinafter, embodiments of the present invention will be described in detail. The present invention is not limited to the following embodiments, and can be variously modified and implemented within the scope of the present invention.

The inorganic particle dispersion of the present invention, particularly the inorganic particle dispersion for a cosmetic, comprises a dispersion medium, an inorganic particle dispersed in the dispersion medium, and a block copolymer containing a hydrophobic segment and a hydrophilic segment, wherein the hydrophobic segment comprises a monomer unit composed of at least one monomer selected from the following Formula 1 and Formula 2, wherein at least a part of the hydrophilic segment is adsorbed on the inorganic particle and the hydrophobic segment is oriented outwardly with respect to the inorganic particle. 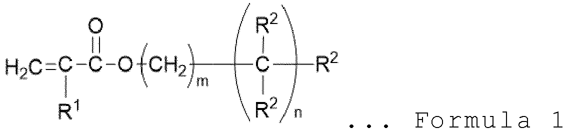
where R¹ is hydrogen or a methyl group,
R² is hydrogen or fluorine,
m is an integer of 0 to 6, and
n is an integer of 1 to 15,

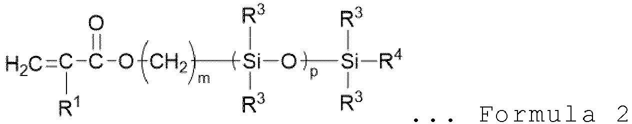

where R¹ is hydrogen or a methyl group,
R³ and R⁴ are each independently an alkyl group having 1 to 6 carbon atoms,
m is an integer of 1 to 6, and
p is an integer of 5 to 70.

Although not limited by the principle, it is considered that the operating principle by which such an inorganic particle dispersion has excellent dispersibility and the like is as follows.

For example, when a random copolymer is prepared from a hydrophobic monomer and a hydrophilic monomer, such a copolymer exhibits an intermediate performance such that hydrophobic and hydrophilic properties are mixed together as a whole of a random copolymer because a hydrophobic site and a hydrophilic site are randomly arranged in the copolymer. On the other hand, in the case of a block copolymer, a hydrophobic segment consisting of a hydrophobic monomer and a hydrophilic segment consisting of a hydrophilic monomer are separately formed in the copolymer, so that the block copolymer can be imparted with a portion having different properties such as hydrophobicity and hydrophilicity, respectively.

For example, when an oil containing inorganic particles surface-treated with a surface treatment agent of a low molecular type conventionally commonly used is blended into a cosmetic or the like and emulsified, aggregation and precipitation tend to occur. This is considered to be due to the fact that a part of the surface treatment agent of such a low molecular type is easily desorbed from the inorganic particle and transferred into the cosmetic, so that a portion which is exposed on the surface of the inorganic particle is generated, and thus, the stability is lacking. On the other hand, the block copolymer of the present invention has a hydrophobic segment and a hydrophilic segment, and at least a part of the hydrophilic segment therein adsorbs on the inorganic particle via a plurality of adsorption points or adsorbs to be entangled on the surface of the inorganic particle, and in some cases, hydrogen bonding or the like occurs between the inorganic particle and the hydrophilic segment, so that it is considered that such a copolymer hardly desorbs from the inorganic particle and improves the dispersion stability of the inorganic particle.

Also, for example, when a hydrophilic segment of a block copolymer is formed from a monomer of Formula 7 above, such a copolymer adsorbs and hydrolyzes on the surface of the inorganic particle to be surface-treated to form a crosslinked structure between at least hydrophilic segments of the copolymer. Since the copolymer having such a crosslinked structure is easily entangled with the inorganic particle, it is considered that the copolymer is hardly desorbed from the inorganic particle. In addition, when the inorganic particle has a hydroxyl group on their surface, a functional group, which is hydrolyzed and subjected to a crosslinking reaction, in the hydrophilic segment reacts with the hydroxyl group on the surface of the inorganic particle and binds thereto. As a result, it is considered that the copolymer becomes more difficult to desorb from the inorganic particle.

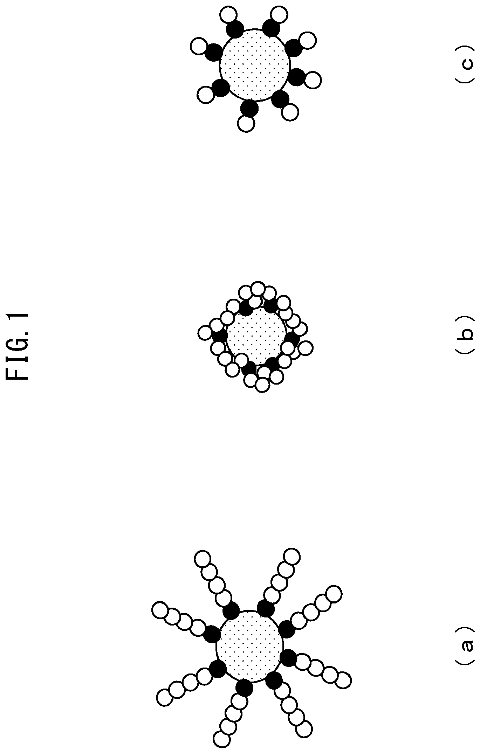

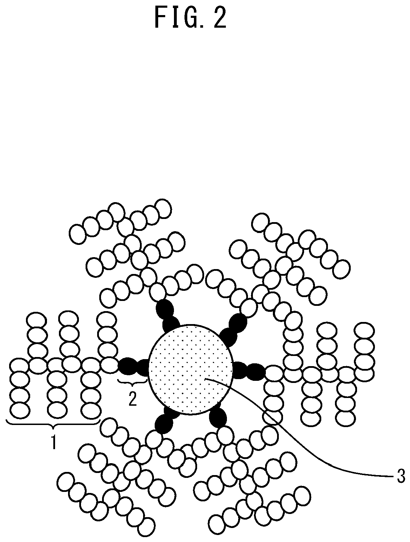

In the oil phase, it is considered that the hydrophobic segment in the copolymer exhibits a comb-shaped structure, for example, as shown in FIG. 2, and is oriented outwardly relative to the inorganic particle. As a result, as shown in FIG. 1(b) and FIG. 1(c), as compared with the surface treatment agents of the random copolymer and the low-molecular type, the steric hindrance action is improved, and the aggregation and precipitation of the inorganic particles in the oil phase can be further suppressed, and thus, it is considered that the dispersibility of the inorganic particles is further improved.

Further, for example, when inorganic particles are blended into a cosmetic of a water-in-oil type, inorganic particles by conventional surface treatment generally tend to greatly increase the viscosity of a cosmetic, but inorganic particles surface-treated with the block copolymer of the present invention do not greatly increase the viscosity of a cosmetic.

It is considered that, for example, an inorganic particle surface-treated with a random copolymer has a structure in which hydrophilic and hydrophobic portions cling to a particle, as shown in FIG. 1(b), and the steric hindrance action is low, and an affinity layer based on the hydrophobic portion between the oil phase and the particle is thin, so that particles are dispersed in an oil phase in close proximity to each other.

Further, it is considered that an inorganic particle surface-treated with a conventional low-molecular type surface treatment agent also has a short hydrophobic portion, as shown in FIG. 1(c), the steric hindrance action is low, and the affinity layer based on the hydrophobic portion between the oil phase and the particle is thin, so that the particles are dispersed in the oil phase in close proximity to each other. As a result, it is considered that the inorganic particles surface-treated with these materials exhibit a state in which the oil is densely packed between the particles, so that the viscosity of a cosmetic or the like increases.

On the other hand, it is considered that the inorganic particles surface-treated by the block copolymer of the present invention have the affinity layer due to the hydrophobic segment between the oil phase and the particle, as shown in FIG. 1(a), in which the affinity layer is covered thicker than the configuration based on the surface treatment agents of the random copolymer and the low-molecular type, and the particles are dispersed relatively apart from each other, so that the oil that exists between the particles can flow more freely. As a result, it is considered that an increase in the viscosity of a cosmetic or the like can be suppressed.

Therefore, for example, when titanium oxide or the like is surface-treated with the block copolymer of the present invention, it is considered that such particles can be highly blended with high dispersibility without greatly increasing the viscosity of cosmetics for sunscreen or the like, so that the performance such as coatability to the skin and sunscreen prevention performance can be improved.

### <<Block copolymer>>

In consideration of dispersibility of inorganic particles, low viscosity such as cosmetics blending inorganic particles, and the like, the block copolymer of the present invention can be set the proportion of the hydrophobic segment to 50 mol% or more, 55 mol% or more, or 60 mol% or more, and can be set to 99 mol% or less, 95 mol% or less, or 90 mol% or less. In consideration of adsorption property and the like to inorganic particles, the proportion of the hydrophilic segment can be set to 1 mol% or more, 5 mol% or more, or 10 mol% or more, and can be set to 50 mol% or less, 45 mol% or less, or 40 mol% or less.

Although there is no particular limitation on the molecular weight of the block copolymer of the present invention, for example, the number average molecular weight in terms of polystyrene in the gel permeation chromatographic measurement can be set in the range of 1000 to 80,000, and is preferably in the range of 5000 to 20,000. Further, as the molecular weight distribution which is a ratio of the number average molecular weight and the weight average molecular weight, it can be set in a range of 1.05 to 5, and is preferably in a range of 1.05 to 1.7.

### <Hydrophobic segment>

The monomer unit composed of at least one monomer selected from Formula 1 and Formula 2 in the hydrophobic segment can be appropriately selected in consideration of affinity and the like with the dispersion medium for blending the inorganic particles.

### (Monomer of Formula 1)

The following monomer of Formula 1 is preferably employed, for example, when a dispersion medium such as a polar oil or a fluorine-based oil is used:

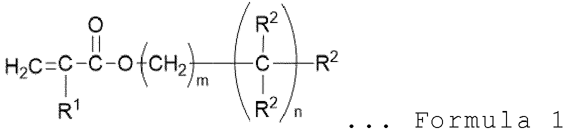

In Formula 1, R¹ is hydrogen or a methyl group, R² is hydrogen or fluorine, m is an integer of 0 to 6, and n is an integer of 1 to 15. From the viewpoint of performance such as dispersibility, low viscosity, and the like, R¹ is preferably a methyl group, and R² is preferably hydrogen or fluorine, and m is preferably an integer of 1 to 3, and n is preferably an integer of 4 or more, 6 or more, or 8 or more, and is preferably an integer of 14 or less, 13 or less, or 12 or less. Here, the sites of (CH₂)ₘ and (CR²₂)ₙ in Formula 1 may be either linear or branched, but is preferably linear.

### (Monomer of Formula 2)

The following monomer of Formula 2 is preferably employed, for example, when a dispersion medium such as a silicone oil is used:

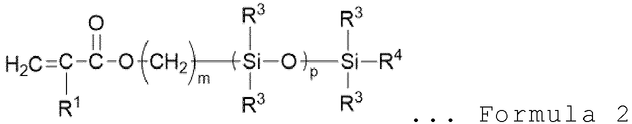

In Formula 2, R¹ is a hydrogen or a methyl group, R³ and R⁴ are each independently an alkyl group having 1 to 6 carbon atoms, m is an integer of 1 to 6, and p is an integer of 5 to 70. From the viewpoint of dispersibility, low viscosity, and the like, R¹ and R³ of Formula 2 are preferably a methyl group, R⁴ is preferably a butyl group, and m is preferably an integer of 1 to 3, p is preferably an integer of 6 or more, 8 or more, or 10 or more, and is preferably an integer of 60 or less, 50 or less, or 40 or less. Here, the site of (CH₂)ₘ in Formula 2 may be either linear or branched, but is preferably linear.

### <Hydrophilic segment>

Since inorganic particles not subjected to surface treatment generally exhibit hydrophilicity on their surface, hydrophilic segments in the block copolymer can be adsorbed on the surface of inorganic particles. Therefore, any monomer constituting such a segment may be used as long as it is a hydrophilic monomer which can be dissolved in water, and is not limited to the following, and for example, a monomer having a hydrophilic group such as represented by a hydroxyl group, an amide group, a sulfate group, a sulfonic acid group, a carboxylic acid group, an oxyethylene group, a pyridine group, or the like can be used.

As the monomer constituting the hydrophilic segment, specifically, for example, at least one monomer selected from Formula 3 to Formula 9 can be used. The monomer unit composed of these monomers can be appropriately selected in consideration of adsorption property and the like with inorganic particles, but among them, a monomer of Formula 3 or Formula 7 is preferred because it can be firmly adsorbed or bonded to inorganic particles.

### (Monomer of Formula 3)

In the monomer of Formula 3 below, R¹ is hydrogen or a methyl group. From the viewpoint of polymerizability and the like of the block copolymer, R¹ is preferably a methyl group:

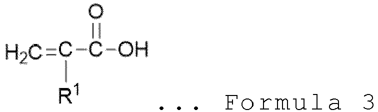

### (Monomer of Formula 4)

In the monomer of Formula 4 below, R¹ is hydrogen or a methyl group, m¹ and m² are each independently integers of 1 to 6, and R⁵ is each independently an alkyl group having 1 to 6 carbon atoms. From the viewpoint of polymerizability of the block copolymer or adsorption of the block copolymer to inorganic particles, and the like, R¹ and R⁵ are preferably methyl groups, and m¹ and m² are each independently preferably an integer of 1 to 3. Here, the sites of (CH₂)ₘ₁ and (CH₂)ₘ₂ site in Formula 4 may be linear or branched, but is preferably linear:

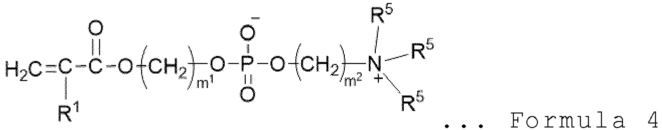

### (Monomer of Formula 5)

In the following monomer of Formula 5, R¹ is hydrogen or a methyl group, m is an integer of 1 to 6, and R⁶ is each independently an alkyl group having 1 to 6 carbon atoms. From the viewpoint of polymerizability of the block copolymer or adsorption of the block copolymer to inorganic particles, and the like, R¹ and R⁶ are preferably methyl groups, and m is preferably an integer of 1 to 3. Here, the site of (CH₂)ₘ in Formula 5 may be either linear or branched, but is preferably linear:

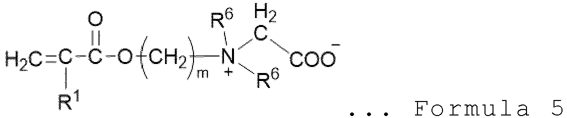

### (Monomer of Formula 6)

In the following monomer of Formula 6, R¹ is hydrogen or a methyl group, m is an integer of 1 to 6, and R⁷ is each independently an alkyl group having 1 to 6 carbon atoms. From the viewpoint of polymerizability of the block copolymer or adsorption of the block copolymer to inorganic particles, and the like, R¹ and R⁷ are preferably methyl groups, and m is preferably an integer of 1 to 3 Here, the site of (CH₂)ₘ in Formula 6 may be either linear or branched, but is preferably linear:

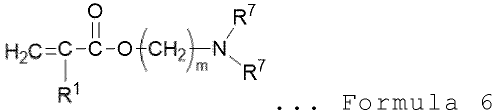

### (Monomer of Formula 7)

In the following monomer of Formula 7, R¹ is hydrogen or a methyl group, m is an integer of 1 to 6, and R⁸ is each independently a functional group which hydrolyzes and crosslinks. From the viewpoint of polymerizability of the block copolymer or adsorption of the block copolymer to inorganic particles, and the like, R¹ is preferably a methyl group, and R⁸ is preferably at least one selected from a hydrogen atom, an alkoxy group, a halogen atom, an acyloxy group and an amino group, and among them, a methoxy group or an ethoxy group is more preferred, and m is preferably an integer of 1 to 3. Here, the site of (CH₂)ₘ in Formula 7 may be either linear or branched, but is preferably linear:

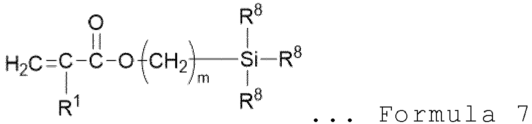

### (Monomer of Formula 8)

In the following monomer of Formula 8, R¹ is hydrogen or a methyl group, q is an integer of 1 to 20, and R⁹ is an alkyl group having 1 to 6 carbon atoms. From the viewpoint of polymerizability and the like of the block copolymer, R¹ is preferably a methyl group:

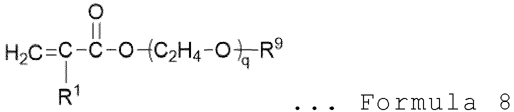

### (Monomer of Formula 9)

The hydrophilic segment of the block copolymer of the present invention may contain a monomer unit composed of the monomer of the following Formula 9

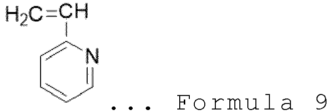

### (Optional monomer)

As long as the effect of the present invention is not impaired, the block copolymer of the present invention may also contain a monomer other than the monomer of the above Formula 1 to Formula 9 as a constituent monomer. The content may be in the range of 30 mol% or less, 20 mol% or less, 10 mol% or less, or 5 mol% or less of the total amount of the constituent monomers. Examples of such monomers include acrylamide, methacrylamide, methyl acrylamide, methyl methacrylamide, dimethyl methacrylamide, ethyl acrylamide, ethyl methacrylamide, diethyl methacrylamide, N-isopropylacrylamide, N-vinylpyrrolidone, ε-caprolactam, vinyl alcohol, maleic anhydride, N,N'-dimethylaminoethyl methacrylic acid, diallyldimethylammonium chloride, alkyl acrylate, alkyl methacrylate, N,N'-dimethylacrylamide, styrene, and the like.

### <Method for producing the block copolymer>

The block copolymer of the present invention can be obtained by a known living radical polymerization method. For example, at least one monomer selected from Formula 1 and Formula 2 described above may be used to form a hydrophobic segment by a living radical polymerization method, and then a hydrophilic segment may be formed by a living radical polymerization method using a hydrophilic monomer to obtain a block copolymer. Alternatively, a hydrophilic monomer is used, a hydrophilic segment is formed by a living radical polymerization method, and then at least one monomer selected from Formula 1 and Formula 2 described above is used, and a hydrophobic segment is formed by a living radical polymerization method to obtain a block copolymer.

The living radical polymerization method is a method in which a catalyst, a chain transfer agent, or the like is added to a conventional radical polymerization method to control the reactivity of a terminal active radical to cause the polymerization to proceed in a pseudo living manner. The molecular weight distribution can be narrowed in comparison with the usual radical polymerization, and the control of the molecular weight is also possible. Specific examples of the known living radical polymerization method include a living radical polymerization method using a non-metal catalyst disclosed in WO 2010/016523 A and the like, a ATRP method by adding a metal complex disclosed in WO 96/030421 A and the like, a TEMPO method for introducing a thermal dissociation group disclosed in US 4,581,429 and the like, a RAFT polymerization method for adding a reversible additional cleavage chain transfer agent disclosed in WO 98/001479 A and the like, and an iniferta method having a photo-thermal dissociation group disclosed in Chem. Express 5 (10), 801 (1990), and the like.

Among them, a living radical polymerization method using a non-metal catalyst which is inexpensive and has little load on the environment is preferred. Such a polymerization method is carried out, for example, using various monomers described above, an initiation compound, a catalyst, a radical polymerization initiator, and a polymerization solvent.

### (Initiation compound)

As the initiation compound, an iodine compound represented by the following Formula 10 is preferably used:

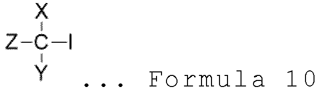

The iodine atom in such an initiation compound is bonded to a secondary or tertiary carbon atom, and X, Y, and Z may be the same or different, and are preferably selected from hydrogen, a hydrocarbon group, a halogen group, a cyano group, an alkoxycarbonyl group, an allyloxycarbonyl group, an acyloxy group, an allyloxy group, an alkoxy group, an alkylcarbonyl group, and an allylcarbonyl group.

It is preferable that the iodine atom is bonded to a secondary or tertiary carbon atom in consideration of the dissociation property of iodine. As a result, at least 2 of X, Y and Z are not hydrogen atoms. Specific examples of X, Y and Z will be described below, but are not limited thereto.

Examples of the hydrocarbon group include an alkyl group, an alkenyl group, an alkynyl group, an aryl group, and an arylalkyl group. Specifically, examples thereof include alkyl groups such as methyl, ethyl, propyl, isopropyl, butyl, isobutyl, 2-methylpropyl, t-butyl, pentyl, dodecyl; alkenyl groups including double bond such as vinyl, allyl, 2-methylvinyl, butenyl, butadienyl; alkynyl groups including triple bond such as acetylene, methylacetylene; aryl groups such as phenyl, naphthyl, methylphenyl, ethylphenyl, propylphenyl, dodecylphenyl, biphenyl, in which the aryl group can include heterocyclic rings such as pyridinyl, imidazolinyl; arylalkyl groups such as phenylmethyl, 1-phenylethyl, 2-phenylethyl, 2-phenylpropyl, and the like.

Examples of the halogen group include a fluorine atom, a chlorine atom, a bromine atom, and an iodine atom.

Examples of the alkoxycarbonyl group or allyloxycarbonyl group include methoxycarbonyl, ethoxycarbonyl, propylcarbonyl, cyclohexylcarbonyl, benzyloxycarbonyl, phenoxycarbonyl, and naphthoxycarbonyl.

Examples of the acyloxy group or the allyloxy group include acetoxy, ethylcarbonyloxy, cyclohexylcarbonyloxy, benzoyloxy, and naphthylcarboxyoxy.

Examples of the alkoxy group include methoxy, ethoxy, methoxyethoxy, and phenoxyethoxy.

Examples of the alkylcarbonyl group or allylcarbonyl group include methylcarbonyl, ethylcarbonyl, and phenylcarbonyl.

Preferred specific examples of the initiation compound include 1-iodo-1-phenylethane, 2-iodo-2-cyanopropane, 2-iodo-2-cyano-4-methylpentane.

In addition, in such a polymerization method, the molecular weight of the copolymer can be controlled by the amount of the initiation compound. By setting the number of moles of the monomer relative to the number of moles of the initiation compound, it is possible to control an arbitrary molecular weight or a large or small molecular weight. The molar ratio of the initiation compound to the monomer is not particularly limited. For example, when 1 mol of an initiation compound is used to polymerize using 500 mol of a monomer having a molecular weight of 100, a theoretical molecular weight of 1 × 100 × 500=50,000 can be provided.

### (Catalyst)

As the catalyst, a nonmetallic compound which becomes a radical capable of abstracting an iodine of an initiation compound, or iodine at a polymer terminal, for example, a phosphorus compound, a nitrogen compound or an oxygen compound, or the like, having such a property, can be used.

Examples of the phosphorus compound include, but are not limited to the following, phosphorus halide containing an iodine atom, a phosphite-based compound, a phosphinate-based compound, and the like, and examples of the nitrogen compound include an imide-based compound, hydantoins, barbituric acids, and cyanuric acids, and examples of the oxygen compound include a phenol-based compound, an iodooxyphenyl compound, and vitamins. These may be used alone or in combination of 2 or more thereof.

Specifically, examples of the phosphorus compound include a halogenated phosphorus containing an iodine atom, a phosphite-based compound, or a phosphinate-based compound, and for example, phosphorus dichloroiodide, phosphorus dibromoiodide, phosphorus triiodide, dimethylphosphite, diethylphosphite, dibutylphosphite, di (perfluoroethyl) phosphinate, diphenylphosphite, dibenzylphosphite, bis (2-ethylhexyl) phosphite, bis (2,2,2-trifluoroethyl) phosphite, diallylphosphite, ethylene phosphite, ethylphenylphosphinate, phenylphenylphosphinate, ethylmethylphosphinate, phenylmethylphosphinate, and the like.

Examples of the nitrogen compound include imides such as succinimide, 2,2-dimethylsuccinimide, α,α-dimethyl-β-methylsuccinimide, 3-ethyl-3-methyl-2,5-pyrrolidinedione, cis-1,2,3,6-tetrahydrophthalimide, α-methyl-α-propylsuccinimide, 5-methylhexahydroisoindole-1,3-dione, 2-phenylsuccinimide, α-methyl-α-phenylsuccinimide, 2,3-diacetoxysuccinimide, maleimide, phthalimide, 4-methylphthalimide, N-chlorophthalimide, N-bromophthalimide, 4-nitrophthalimide, 2,3-naphthalenecarboxyimide, pyromellitodiimide, 5-bromoisoindole-1,3-dione, N-chlorosuccinimide, N-bromosuccinimide, N-iodosuccinimide, and the like. Examples of the hydantoins include hydantoin, 1-methylhydantoin, 5,5-dimethylhydantoin, 5-phenylhydantoin, 1,3-diiodo-5,5-dimethylhydantoin, and the like. Examples of the barbituric acids include barbituric acid, 5-methylbarbituric acid, 5,5-diethylbarbituric acid, 5-isopropylbarbituric acid, 5,5-dibutylbarbituric acid, thiobarbituric acid, and the like. Examples of the cyanuric acids include cyanuric acid, N-methylcyanuric acid, triiodocyanuric acid, and the like.

Examples of the oxygen compound include a phenol-based compound which has a phenolic hydroxyl group with a hydroxyl group at an aromatic ring, an iodooxyphenyl compound which is an iodide of the phenolic hydroxyl group thereof, and vitamins. Examples of the phenols include phenol, hydroquinone, 4-methoxyphenol, 4-t-butylphenol, 4-t-butyl-2-methylphenol, 2-t-butyl-4-methylphenol, catechol, resorcin, 2,6-di-t-butyl-4-methylphenol, 2,6-dimethylphenol, 2,4,6-trimethylphenol, 2,6-di-t-butyl-4-methoxyphenol, polymeric microparticles supported with a polymer that is polymerized with 4-hydroxystyrene, or supported with its hydroxyphenyl group, a monomer having a phenolic hydroxyl group such as methacrylic acid 3,5-di-t-butyl-4-hydroxyphenylethyl. Since these are added as polymerization inhibitors in an ethylenically unsaturated monomer, it is also possible to exert an effect by using an ethylenically unsaturated monomer of a commercially available product as it is without purification. Examples of the iodooxyphenyl compound include thymol iodide and the like, and examples of the vitamins include vitamin C and vitamin E.

The amount of the catalyst to be used is generally less than the number of moles of the radical polymerization initiator, and may be arbitrarily determined in consideration of the control state of the polymerization and the like.

### (Radical polymerization initiator)

As the radical polymerization initiator, conventionally known ones can be used, and there is no particular limitation, and for example, an organic peroxide or an azo compound or the like can be used. Specifically, examples thereof include benzoyl peroxide, dicumyl peroxide, diisopropyl peroxide, di-t-butyl peroxide, t-butyl peroxybenzoate, t-hexyl peroxybenzoate, t-butyl peroxy-2-ethylhexanoate, t-hexylperoxy-2-ethylhexanoate, 1,1-bis(t-butyl peroxy)3,3,5-trimethylcyclohexane, 2,5-dimethyl-2,5-bis(t-butylperoxy)hexyl-3,3-isopropylhydroperoxide, t-butyl hydroperoxide, dicumyl hydroperoxide, acetyl peroxide, bis(4-t-butylcyclohexyl)peroxydicarbonate, isobutyl peroxide, 3,3,5-trimethylhexanoylperoxide, lauryl peroxide, 1,1-bis(t-butylperoxy)3,3,5-trimethylcyclohexane, 1,1-bis(t-hexylperoxy)3,3,5-trimethylcyclohexane, 2,2'-azobis(isobutyronitrile), 2,2'-azobis(2,4-dimethylvaleronitrile), 2,2'-azobis(4-methoxy-2,4-dimethylvaleronitrile), dimethyl 2,2'-azobis(isobutyrate), and the like.

From the viewpoint of polymerizability and the like, the radical polymerization initiator can be used in a range of 0.001 mol times or more, 0.002 mol times or more, or 0.005 mol times or more, based on the number of moles of the monomer, and can be used in a range of 0.1 mol times or less, 0.05 mol times or less, or 0.01 mol or less.

### (Polymerization solvent)

As the polymerization solvent, a solvent which does not exhibit reactivity with respect to the functional group of the monomer is appropriately selected. Examples thereof include, but not limited to the following, hydrocarbonic solvents such as hexane, octane, decane, isodecane, cyclohexane, methylcyclohexane, toluene, xylene, ethylbenzene, cumene; alcoholic solvents such as methanol, ethanol, propanol, isopropanol, butanol, isobutanol, hexanol, benzyl alcohol, cyclohexanol; hydroxyl-containing glycol ethers such as ethylene glycol, diethylene glycol, propylene glycol, dipropylene glycol, methyl cellosolve, ethyl cellosolve, butyl cellosolve, propylene glycol monomethyl ether, propylene glycol monoethyl ether, propylene glycol propyl ether, butylcarbitol, butyltriethylene glycol, methyldipropylene glycol; glycolic solvents such as diglyme, triglyme, methyl cellosolve acetate, propylene glycol monomethyl ether acetate, dipropylene glycol butyl ether acetate, diethylene glycol monobutyl ether acetate; etheric solvents such as diethyl ether, dimethyl ether, dipropyl ether, methylcyclopropyl ether, tetrahydrofuran, dioxane, anisole; ketonic solvents such as dimethyl ketone, diethyl ketone, ethyl methyl ketone, isobutyl methyl ketone, cyclohexanone, isophorone, acetophenone; ester solvents such as methyl acetate, ethyl acetate, butyl acetate, propyl acetate, methyl butyrate, ethyl butyrate , caprolactone, methyl lactate, ethyl lactate; halogenated solvents such as chloroform, dichloromethane, dichloroethane, o-dichlorobenzene; amide-based solvents such as formamide, N,N-dimethylformamide, N,N-dimethylacetamide, 2-pyrrolidone, N-methyl-2-pyrrolidone, ε-caprolactam; dimethyl sulfoxide, sulfolane, tetramethylurea, ethylene carbonate, propylene carbonate, dimethyl carbonate, diethyl carbonate, nitromethane, acetonitrile, nitrobenzene, dioctylphthalate, and the like.

### (Polymerization temperature)

The polymerization temperature is appropriately adjusted by the half-life of the radical polymerization initiator, and is not particularly limited, and may be, for example, 0 °C or more or 30 °C or more, and may be 150 °C or less or 120 °C or less.

### (Polymerization time)

It is preferable to continue polymerization until the monomer is gone, and the polymerization time is not particularly limited, and may be, for example, 0.5 hours or more, 1 hours or more, or 2 hours or more, and may be 48 hours or less, 24 hours or less, or 12 hours or less.

### (Polymerization atmosphere)

The polymerization atmosphere is not particularly limited, and may be polymerized as it is under an atmospheric atmosphere, that is, oxygen may be present within a normal range in the polymerization system, or may be carried out under a nitrogen atmosphere in order to remove oxygen if necessary. Impurities may be removed from various materials to be used by distillation, activated carbon, alumina, or the like, but a commercially available product may be used as it is. Also, the polymerization may be carried out under light shielding and may be carried out in a transparent container such as glass.

### <<Inorganic particle dispersion>>

The block copolymer of the present invention can be used as a surface treatment agent for inorganic particles. The surface treatment of the inorganic particles by the block copolymer of the present invention may be performed using an ordinary treatment method, and the method is not particularly limited. For example, the block copolymer of the present invention may be dissolved in a suitable dispersion medium, and inorganic particles may be mixed and stirred in this solution to obtain a dispersion containing surface-treated inorganic particles. The surface-treated inorganic particles may be used in the form of a dispersion, or may be used in the form of a dry powder.

When the block copolymer of the present invention is applied to inorganic particles as a surface treatment agent, the proportion of the inorganic particles and the block copolymer is not particularly limited as long as the desired performance such as dispersibility is exhibited, but may be, for example, in the range of 100:5 to 100:40 in a mass ratio, and is preferably in the range of 100:7 to 100:30, and more preferably in the range of 100:10 to 100:20.

Further, the content of the inorganic particles in the dispersion can be set in a range of 10% by mass or more, 20% by mass or more, or 30% by mass or more of the entire dispersion, and can be set in a range of 90% by mass or less, 80% by mass or less, or 70% by mass or less.

### <Inorganic particles>

The inorganic particles are not particularly limited and may be used alone or in combination of 2 or more, and inorganic particles having a hydrophilic surface, among them, inorganic particles having hydroxyl groups on their surfaces, for example, metal oxides, are preferred. Such hydroxyl groups on the surface of the inorganic particles may form hydrogen bonds or the like with the hydrophilic segments. Such inorganic particles are not particularly limited, and examples thereof include particles such as silicic acid, silicic anhydride, magnesium silicate, talc, kaolin, mica, bentonite, titanium coated mica, bismuth oxychloride, zirconium oxide, magnesium oxide, titanium oxide, zinc oxide, cerium oxide, iron oxide, aluminum oxide, calcium sulfate, barium sulfate, magnesium sulfate, calcium carbonate, magnesium carbonate, ultramarine blue, iron blue, chromium oxide, chromium hydroxide, carbon black, and composites thereof. Also for the shape of the particles, for example, plate-like, massive, scaly, spherical, porous spherical, etc., can be used in any shape, it is not particularly limited for particle size.

### (Dispersion medium)

Organic solvents, especially various oils can be used as a dispersion medium. Examples thereof include, are not limited to the following, hydrocarbon oils such as liquid paraffin, squalane, isoparaffin, branched chain light paraffin, petrolatum, and seresin; ester oils such as isopropyl myristate, cetyl isooctanoate, and glyceryl trioctanoate; and silicone oils such as decamethylpentasiloxane, dimethylpolysiloxane, and methylphenylpolysiloxane. These may be used alone or in combination of 2 or more thereof.

Among them, silicone oil is suitably used from the viewpoint of feeling of use and the like when used as a cosmetic or the like. Specifically, based on the entire dispersion medium, the silicone oil can be used in a range of 10% by mass or more, 50% by mass or more, or 70% by mass or more, and can be used in a range of 100% by mass or less.

The silicone oil is not limited to the following, and for example, a chain polysiloxane, a cyclic polysiloxane, a modified silicone, a silicone-based resin, or the like can be used, but particularly, a silicone oil having a boiling point of 200 °C or less at ordinary pressure is suitable. Examples thereof include chain polysiloxanes such as dimethylpolysiloxane, methylphenylpolysiloxane, and methylhydrogenpolysiloxane; and cyclic polysiloxanes such as octamethylcyclotetrasiloxane, decamethylcyclopentasiloxane, dodecamethylcyclohexasiloxane, and tetramethyltetrahydrogencyclotetrasiloxane.

Among them, when a volatile silicone oil such as a volatile chain polysiloxane such as a low degree of polymerization dimethylpolysiloxane (degree of polymerization 3 to 7) or a cyclic volatile polysiloxane such as decamethylcyclopentasiloxane or octamethylcyclotetrasiloxane is used, it is particularly suitable because, for example, when applied to the skin as a cosmetic or the like, an oil feeling hardly remains and a refreshing feeling of use is obtained.

### <<Applications of inorganic particle dispersions>>

Since the dispersion of the inorganic particles surface-treated with the block copolymer of the present invention can greatly suppress the increase in viscosity accompanying the blending of the inorganic particles as compared with the inorganic particle dispersion system by the conventional surface treatment, it is possible to highly blend the inorganic particles without limiting the formulation. Therefore, such a dispersion can be used in various applications, for example, a cosmetic, a resin composition, a paint, an ink, a composition for coating, and the like, and among them, it is preferable to use it in a cosmetic, particularly a cosmetic for sunscreen. Although the present invention is not limited, specifically, a cosmetic using a dispersion of inorganic particles surface-treated with the block copolymer of the present invention will be described below.

### <Cosmetics>

When the inorganic particle dispersion of the present invention is applied to a cosmetic, it can be made into an oily type cosmetic as it is or diluted with an oily component, and further, these can be emulsified with an aqueous phase component by a known method to obtain an oil-in-water type or water-in-oil type emulsified cosmetic, particularly an oil-in-water type emulsified cosmetic.

In a cosmetic containing the inorganic particle dispersion of the present invention, particularly a cosmetic for sunscreen, an ultraviolet scattering agent such as titanium oxide, zinc oxide, cerium oxide, iron oxide, or mica can be highly blended in a cosmetic with good dispersibility without greatly increasing viscosity. As a result, such a cosmetic is excellent in applicability to skin, and also can provide an increase in SPF value based on scattering and shielding effect of ultraviolet rays by a uniformly dispersed scattering agent.

Since the SPF value is a numerical value which varies depending on the degree of dispersion of the inorganic particles, it is also possible to use the SPF value as an indicator of dispersion stability. The SPF value and viscosity of such a cosmetic are not limited to the following. The SPF value may be, for example, 15 or more, 20 or more, or 25 or more, 60 or less, 55 or less, or 50 or less, and the viscosity may be 50000Pa·s or less, 30000Pa·s or less, or 10000Pa·s or less, particularly, may be 3000Pa·s or less, 2800Pa·s or less, or 2500Pa·s or less, and may be 100Pa·s or more, 150Pa·s or more, or 200Pa·s or more. Here, the viscosity means a viscosity at a shear rate of 1/s of an object to be measured when measured at 32 °C and 1 atm using VDA-2 or VS-H1 (both manufactured by Shibaura System Co., Ltd.) as a bistometron viscometer.

The inorganic particle dispersion of the present invention can highly blend inorganic particles in a cosmetic, and is not limited to the following, and for example, the amount of the inorganic particles to be blended in the cosmetic can be 5% by mass or more, 10% by mass or more, or 15% by mass or more based on the total amount of the cosmetic, and can be 50% by mass or less, 48% by mass or less, or 45% by mass or less.

The dosage form of the cosmetic of the present invention is optional and can be provided in any form, such as a solution system, a solubilization system, an emulsification system, a water-oil bilayer system, a gel, an aerosol, a mist, and a capsule. The product form of the cosmetic of the present invention is also optional, and can be applied in any form as long as it is conventionally used for external skin preparations such as facial cosmetics such as cosmetic waters, emulsions, creams, packs, etc.; makeup cosmetics such as cosmetic bases, foundations, blushes, lip sticks, lip creams, eye shadows, eye liners, mascaras, sunscreens, etc.; body cosmetics; aromatic cosmetics; skin cleansers such as makeup removers, face washes, body shampoos, etc.; hair cosmetics such as hair sprays, hair creams, hair lotions, hair rinses, shampoos, etc. In particular, it is suitably used as a product intended for preventing ultraviolet rays.

### (Optional components)

In the cosmetic of the present invention, various components can be appropriately blended within a range that does not affect the effect of the present invention. Various components may be additive components which can usually be blended into cosmetics, for example, liquid fats, solid fats, waxes, oils such as higher fatty acids, higher alcohols, anionic surfactants, cationic surfactants, amphoteric surfactants, nonionic surfactants, moisturizers, water-soluble polymers, thickeners, film agents, sequestering agents, lower alcohols, polyhydric alcohols, various extracts, sugars, amino acids, organic amines, polymer emulsions, chelating agents, ultraviolet absorbers, pH adjusting agents, skin nutritional agents, vitamins, pharmaceuticals, quasi-drugs, water-soluble drugs applicable to cosmetics or the like, antioxidants, buffers, preservatives, antioxidant auxiliary agents, organic-based powders, pigments, dyes, colorants, perfumes, water and the like.

When a cosmetic for sunscreen is used, 1 or 2 or more kinds of water-soluble or oil-soluble organic ultraviolet absorbers may be blended.

Examples of the water-soluble ultraviolet absorber include benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, 4-hydroxy-3-carboxybenzophenone; benzimidazole-based ultraviolet absorbers such as phenylbenzimidazole-5-sulfonic acid and its salts, phenylene-bis-benzimidazole-tetrasulfonic acid and its salts; 3-(4'-methylbenzylidene)-d,1-camphor, 3-benzylidene-d,1-camphor, urocanic acid, and urocanic acid ethyl ester.

Examples of the oil-soluble ultraviolet absorber include benzoic acid-based ultraviolet absorbers such as para-aminobenzoic acid (PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester; anthranilate-based ultraviolet absorbers such as homomentyl-N-acetyl anthranilate; salicylic acid-based ultraviolet absorbers such as amylsalicylate, menthyl salicylate, homomenthyl salisilate, octyl salicylate, phenyl salicylate, benzyl salicylate, p-isopropanolphenyl salicylate; cinnamic acid-based ultraviolet absorbers such as octylcinnamate, ethyl-4-isopropylcinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate, 2-ethylhexyl-p-methoxycinnamate, 2-ethoxyethyl-p-methoxycinnamate, cyclohexyl-p-methoxycinnamate, ethyl-α-cyano-β-phenylsinnamate, 2-ethylhexyl-α-cyano-β-phenylsinnamate, glyceryl-mono-2-ethylhexanoyl-diparamethoxycinnamate, 3,4,5-trimethoxycinnamic acid 3-methyl-4-[methylbis(trimethylsiloxy)silyl]butyl; 2-phenyl-5-methylbenzoxazole, 2,2'-hydroxy-5-methylphenylbenzotriazole, 2-(2'-hydroxy-5'-t-octylphenyl) benzotriazole, 2-(2'-hydroxy-5'-methylphenylbenzotriazole, dibenzalazine, dianisoylmethane, 4-methoxy-4'-t-butyl dibenzoylmethane, 5-(3,3-dimethyl-2-norbornilidene)-3-pentan-2-one, octocrylene.

### EXAMPLES

Hereinafter, the present invention will be described in more detail with reference to Examples, but the present invention is not limited thereto. Note that, hereinafter, unless otherwise specified, the blending amount is shown in parts by mass.

### <<Examples 1 to 5 and Comparative Examples 1 to 8>>

### (Method for synthesizing copolymers)

### (Copolymer 1)

30 parts by mass of propylene glycol monomethyl ether acetate, 110 parts by mass of monomer of Formula 2 below, 0.25 parts by mass of iodine, and 0.3 parts by mass of 2,2'-azobis(isobutyronitrile) were charged into a reaction vessel equipped with a stirrer, a reflux condenser, a thermometer, and a nitrogen inlet tube, and the mixture was polymerized at 80°C for 2 hours while bubbling nitrogen to prepare a hydrophobic segment. 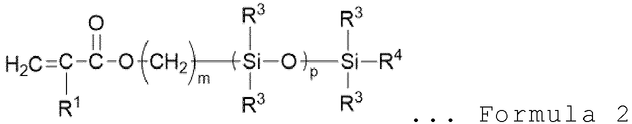 where, R¹ and R³ are methyl groups, R⁴ is a butyl group, m is 3, and p is 10.

Subsequently, 15 parts by mass of methacrylic acid was added and further polymerized at the same temperature for 2 hours to form a hydrophilic block portion to obtain a block copolymer having the hydrophobic segment and the hydrophilic segment. From the amount of each monomer raw material to be used, when the molar ratio of the obtained block copolymer was converted, the proportion of the monomer unit of Formula 2 was about 80.5 mol%, and the proportion of the monomer unit of methacrylic acid was about 19.5 mol%.

### (Copolymer 2)

The block copolymer of copolymer 2 was prepared in the same manner as in copolymer 1, except that p of the monomer of Formula 2 above was changed from 10 to 30.

### (Copolymer 3)

The block copolymer of copolymer 3 was prepared in the same manner as in copolymer 1, except that 80 parts by mass of the monomer of Formula 1 below was used instead of the monomer of Formula 2 above. 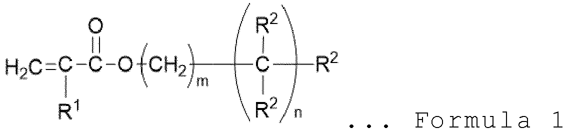 where R¹ is a methyl group, R² is hydrogen, and the sum of m and n is 11.

### (Copolymer 4)

The block copolymer of copolymer 4 was prepared in the same manner as in copolymer 1, except that the monomer of Formula 2 above was replaced with methyl methacrylate.

### <Method for preparing surface-treated inorganic particle dispersion>

### (Dispersion 1)

20 g of copolymer 1 was dissolved in 200g of butyl diglycol, and then 80g of titanium oxide (ST-485WD, manufactured by Titanium Industry Co., Ltd.) was added into this solution and stirred at room temperature for 1 hours to prepare dispersion 1 of titanium oxide containing 50% by mass of surface-treated titanium oxide.

### (Dispersions 2 to 4).

Dispersions 2 to 4 of titanium oxide were prepared in the same manner as in dispersion 1, except that copolymers 2 to 4 were used instead of copolymer 1, respectively.

### (Disperse 5)

Dispersion 5 of zinc oxide was prepared in the same manner as in dispersion 1, except that copolymer 2 and zinc oxide were used instead of copolymer 1 and titanium oxide.

### <Evaluation of viscosity and SPF value>

The viscosity and SPF value, also called ultraviolet protection indices, of emulsions obtained from the formulations and manufacturing methods shown in Tables 1 to 2 below were evaluated. Here, the viscosity at a shear rate of 1/s of an object to be measured when measured at 32 °C and 1 atm was adopted using a VDA-2 in the case of low viscosity and a VS-H1 in the case of high viscosity, as a bistometron viscometer. Further, the SPF value was obtained by applying the obtained emulsion on a transparent tape with a coating amount of 2mg/cm² to obtain an evaluation sample, and such an evaluation sample was inserted between a solar simulator manufactured by Solar Light Co., Ltd., which is a light source having a spectrum substantially the same as that of sunlight in an ultraviolet region, and a spectrophotometer, and a spectrum according to the presence or absence of the evaluation sample was compared to calculate the SPF value. The calculation method from each spectrum is the same as the method described in paragraphs [0076] and [0077] of JP-H06-27064 B.

### <Methods for preparing emulsions of Examples 1 to 2 and Comparative Examples 1 to 6>

### (Example 1)

Cyclopentasiloxane as an oil, and titanium oxide powder obtained by drying dispersion 1 of titanium oxide was stirred and mixed at room temperature to prepare mixture A. Then, PEG-10 dimethicone as a surfactant and ion-exchanged water were stirred and mixed at 60 °C to prepare mixture B, and mixture A was added to such mixture B while stirring at 60 °C to prepare the emulsion of Example 1. Also, the composition of such an emulsion is summarized in Table 1.

### (Example 2)

The emulsion of Example 2 was prepared in the same manner as in Example 1, except that cyclopentasiloxane and cetyl isooctanoate were used as an oil. Also, the composition of such an emulsion is summarized in Table 1

### (Comparative Examples 1 to 2, 4 and 5)

The emulsions of comparative examples 1 to 2, 4 and 5 were produced in the same manner as in Example 1, except that the various components and blending ratios described in Table 1 were adopted, and dispersions B and C were used without being dried. Also, the composition of such emulsions is summarized in Table 1. Here, powder A in Table 1 is a titanium oxide powder surface-treated with aluminum stearate, and dispersion B is a dispersion containing 40% by mass of titanium oxide surface-treated with aluminum stearate and 10% by mass of a surfactant in a cyclopentasiloxane, and dispersion C is a dispersion containing 40% by mass of titanium oxide surface-treated with hydrogen dimethicone and 10% by mass of a surfactant in a cyclopentasiloxane.

### (Comparative Examples 3 and 6)

The emulsions of Comparative Examples 3 and 6 were prepared by stirring and mixing cyclopentasiloxane as an oil, ion-exchanged water, and dispersion B or dispersion C at 60 °C. Also, the composition of such emulsions is summarized in Table 1.

**[Table 1]**

| | | Comparati ve Example 1 | Comparati ve Example 2 | Comparati ve Example 3 | Comparati ve Example 4 | Comparati ve Example 5 | Comparati ve Example 6 | Examp le 1 | Examp le 2 |
|---|---|---|---|---|---|---|---|---|---|
| Titanium oxide | Powder A | 20 | - | - | - | - | - | - | - |
| | Dispersion B | - | 50 | 50 | 50 | - | - | - | - |
| | Dispersion C | - | - | - | - | 50 | 50 | - | - |
| | Dry powder of dispersion 1 | - | - | - | - | - | - | 20 | 20 |
| Oil | Cyclopentasilox ane | 30 | 5 | 5 | - | 5 | 5 | 30 | 25 |
| | Cetyl isooctanoate | - | - | - | 5 | - | - | - | 5 |
| Surfacta nt | PEG-10 dimethicone | 2 | 2 | 0 | 2 | 2 | 0 | 2 | 2 |
| Water | Ion-exchanged water | 48 | 43 | 45 | 43 | 43 | 45 | 48 | 48 |
| Total | | 100 | 100 | 100 | 100 | 100 | 100 | 100 | 100 |
| Viscosity(Pa·s) | | 42100 | 6680 | 7570 | 10650 | Separatio n | 50000 | 1020 | 1100 |

### <Results>

As is apparent from Table 1, it has been found that the emulsions of Comparative Examples 1 to 6 in which titanium oxide surface-treated with a conventional, commonly used material is employed have a high viscosity of about 6700Pa·s or more, or are unstable due to separation of oil and water, but Examples 1 and 2 in which titanium oxide surface-treated with the block copolymer of the present invention is employed have excellent dispersibility of titanium oxide, and in addition, the viscosity of the emulsions can be greatly reduced as compared with Comparative Examples 1 to 6. Further, for example, as can be seen from Comparative Examples 2 and 4, when titanium oxide surface-treated with a conventional material is used, the viscosity varies greatly depending on the type of oil, but it has been found that the emulsions of Examples 1 and 2 are hardly affected by the type of oil. Therefore, it has also been found that an object such as titanium oxide surface-treated with the block copolymer of the present invention is less susceptible to restrictions on the type of oil.

### <Methods for preparing emulsions of Examples 3 to 5 and Comparative Examples 7 to 8>

### (Example 3)

The oil, and dry powders of dispersion 2 which are UV scattering agent, described in Table 2, were stirred and mixed under an atmosphere of 80 °C at the blending ratio described in Table 2 to prepare a mixture C. Then, the water, alcohol, thickener, humectant, surfactant, UV absorber and other components described in Table 2 were stirred and mixed under an atmosphere of 80 °C at the blending ratio described in Table 2 to prepare a mixture D, and while stirring at 80 °C, mixture C was added to such mixture D to prepare the emulsion of Example 3. Also, the composition of such an emulsion is summarized in Table 2.

### (Examples 4 and 5)

Each of the emulsions of Examples 4 and 5 was prepared in the same manner as in Example 3, except that the UV scattering agent was changed from the dry powder of dispersion 2 to the dry powder of dispersion 3 or the dry powder of dispersion 4. Also, the composition of such emulsions is summarized in Table 2

### (Comparative Example 7)

The water, alcohol, thickener, moisturizer, oil, surfactant, UV absorber and other components described in Table 2 were stirred and mixed under an atmosphere of 80 °C at the blending ratio described in Table 2 to prepare the emulsion of Comparative Example 7. Also, the composition of such an emulsion is summarized in Table 2.

### (Comparative Example 8)

The emulsion of Comparative Example 8 was prepared in the same manner as in Example 3, except that the UV scattering agent was changed from the dry powder of dispersion 2 to Powder A. Also, the composition of such an emulsion is summarized in Table 2.

**[Table 2]**

| | | Comparative Example 7 | Comparative Example 8 | Example 3 | Example 4 | Example 5 |
|---|---|---|---|---|---|---|
| Water | Ion-exchanged water | 53.43 | 53.43 | 53.43 | 53.43 | 53.43 |
| Alcohol | 95% ethyl alcohol | 10 | 10 | 10 | 10 | 10 |
| Thickener | Carbomer | 0.05 | 0.05 | 0.05 | 0.05 | 0.05 |
| | (acrylate/alkyl acrylate (C10 to 30)) cross polymer | 0.08 | 0.08 | 0.08 | 0.08 | 0.08 |
| | Xanthan gum | 0.05 | 0.05 | 0.05 | 0.05 | 0.05 |
| Moisturizer | Glycerin | 0.5 | 0.5 | 0.5 | 0.5 | 0.5 |
| Oil | Dimethicone | 10.7 | 10.7 | 10.7 | 10.7 | 10.7 |
| Surfactant | Lauryl betaine | 0.1 | 0.1 | 0.1 | 0.1 | 0.1 |
| UV sorbent | Octyl methoxycinnamate | 11 | 5.5 | 5.5 | 5.5 | 5.5 |
| | Polysilicone-15 | 8.5 | 4.25 | 4.25 | 4.25 | 4.25 |
| UV scattering agent | Powder A | - | 9.75 | - | - | - |
| | Dry powder of dispersion 2 | - | - | 9.75 | - | - |
| | Dry powder of dispersion 3 | - | - | - | 9.75 | - |
| | Dry powder of dispersion 4 | - | - | - | - | 9.75 |
| Other components | Silica | 5 | 5 | 5 | 5 | 5 |
| | Phenoxyethanol | 0.5 | 0.5 | 0.5 | 0.5 | 0.5 |
| | Potassium hydroxide | 0.07 | 0.07 | 0.07 | 0.07 | 0.07 |
| | EDTA-3Na ·2H₂O | 0.02 | 0.02 | 0.02 | 0.02 | 0.02 |
| Total | | 100 | 100 | 100 | 100 | 100 |
| Viscosity(Pa·s) | | 9050 | 22500 or more | 2250 | 2200 | 2500 |
| SPF value | | 5.4 | 3.4 | 7.8 | 7.8 | 7.8 |

### <Results>

As is apparent from Table 2, when a portion of the UV absorber of Comparative Example 7 containing no UV scattering agent is replaced with a UV scattering agent, it is generally accompanied by a large increase in viscosity as shown in Comparative Example 8, and the SPF value tends to decrease due to a decrease in dispersibility of the UV scattering agent. On the other hand, in the case of Examples 3 to 5 in which titanium oxide surface-treated with the block copolymer of the present invention is employed, it has been found that, even if a part of the UV absorber is replaced with a UV scattering agent, the viscosity can be reduced on the contrary rather than the viscosity increases, and also the SPF value can be increased.

In addition, from the results of Comparative Example 7 and Examples 3 to 5 in Table 2, when a UV scattering agent containing titanium oxide or the like surface-treated with the block copolymer of the present invention is used, the SPF value equal to or higher than that of a system containing no UV scattering agent as shown in Comparative Example 7 is obtained in a state of lower viscosity, and therefore, it is considered that it can be easily inferred that when a UV scattering agent is blended at a higher concentration to a viscosity of a system containing no UV scattering agent, the SPF value can be further increased than that of such a system.

### <<Formulation example of inorganic particle dispersion>>

Hereinafter, the formulation example of the inorganic particle dispersion of the present invention will be described, but the present invention is not limited to this illustration. Note that the viscosity immediately after preparation and after 1 days in the milky lotion described in the following formulation example was as low as 660Pa·s and 580Pa·s, and the SPF value achieved 36. It is considered that such results suggest that cosmetics obtained using the inorganic particle dispersion of the present invention can realize both a freshness based on low viscosity and a high SPF value.

**<Formulation Example 1 Milky lotion>**

| (Components) | (Parts by mass) |
|---|---|
| 1. Ion-exchanged water | 21 |
| 2. Cyclopentasiloxane | 27.65 |
| 3. Hydrogenated polydecene | 2 |
| 4. PEG-9 polydimethylsiloxyethyl dimethicone | 3 |
| 5. Isostearic acid | 0.3 |
| 6. Triethylhexanoin | 7 |
| 7. Disteardimonium hectorite | 0.5 |
| 8. Dry powder of dispersion 2 | 12 |
| 9. Dry powder of dispersion 5 | 16 |
| 10. EDTA-3Na ·2H₂O | 0. 2 |
| 11. Xylitol | 1 |
| 12. Glycerin | 4 |
| 13. 1,3 Butanediol | 5 |
| 14. Phenoxyethanol | 0.35 |

### (Method for producing milky lotion)

Mixture E was prepared by stirring and mixing No. 3 and No. 5 to No. 9 above at room temperature. Mixture F was then prepared by stirring and mixing No. 1, No. 2, No. 4 and No. 10 to No. 14 above at room temperature, and mixture E was added to such mixture F while stirring at room temperature to prepare a milky lotion.

### REFERENCE SIGNS LIST

- 1: Hydrophobic segment
- 2: Hydrophilic segment
- 3: Inorganic particle

## Claims

1. An inorganic particle dispersion for a cosmetic comprising a dispersion medium, an inorganic particle dispersed in the dispersion medium, and a block copolymer comprising a hydrophobic segment and a hydrophilic segment,
wherein the hydrophobic segment comprises a monomer unit composed of at least one monomer selected from the following Formula 1 and Formula 2,
wherein at least a portion of the hydrophilic segment is adsorbed on the inorganic particle, and the hydrophobic segment is oriented outwardly relative to the inorganic particle: 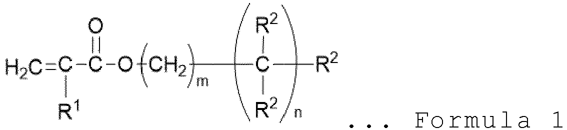
where R¹ is hydrogen or a methyl group,
R² is hydrogen or fluorine,
m is an integer of 0 to 6, and
n is an integer of 1 to 15, 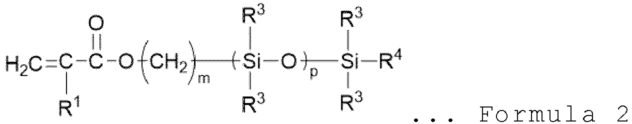
where R¹ is hydrogen or a methyl group,
R³ and R⁴ are each independently an alkyl group having 1 to 6 carbon atoms,
m is an integer of 1 to 6, and
p is an integer of 5 to 70.

2. The dispersion according to claim 1, wherein the hydrophilic segment comprises a monomer unit composed of at least one monomer selected from Formula 3 to Formula 9 below. 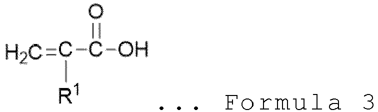 where R¹ is hydrogen or a methyl group, 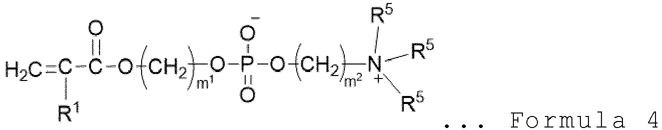
where R¹ is hydrogen or a methyl group,
m¹ and m² are each independently integers of 1 to 6 and
R⁵ is each independently an alkyl group having 1 to 6 carbon atoms, 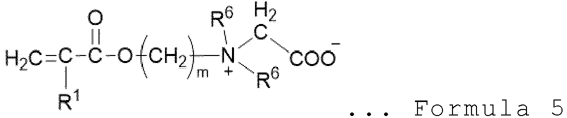
where R¹ is hydrogen or a methyl group,
m is an integer of 1 to 6, and
R⁶ is each independently an alkyl group having 1 to 6 carbon atoms, 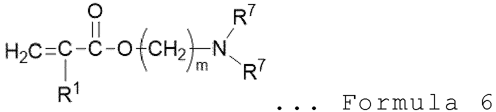
where R¹ is hydrogen or a methyl group,
m is an integer of 1 to 6, and
R⁷ is each independently an alkyl group having 1 to 6 carbon atoms, 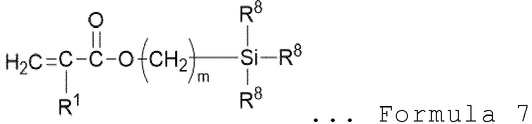
where R¹ is hydrogen or a methyl group,
m is an integer of 1 to 6, and
R⁸ is each independently a functional group that hydrolyzes and cross-links, 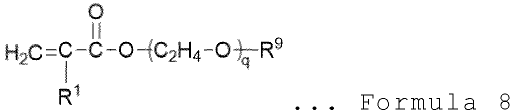
where R¹ is hydrogen or a methyl group,
q is an integer of 1 to 20, and
R⁹ is an alkyl group having 1 to 6 carbon atoms,

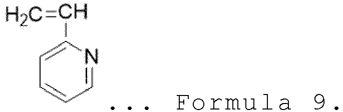

3. The dispersion according to claim 1 or 2, wherein in the block copolymer, the proportion of the hydrophobic segment is from 50 to 99 mol% and the proportion of the hydrophilic segment is from 1 to 50 mol%.

4. The dispersion according to any one of claims 1 to 3, wherein the proportion of the inorganic particle and the block copolymer is from 100:5 to 100:40 by mass.

5. The dispersion according to any one of claims 1 to 4, wherein the inorganic particle is at least one selected from a titanium oxide particle, a zinc oxide particle, a cerium oxide particle, an iron oxide particle, and a mica particle.

6. An inorganic particle powder, wherein the dispersion according to any one of claims 1 to 5 is dried.

7. A sunscreen cosmetic comprising the dispersion according to any one of claims 1 to 5 or the inorganic particle powder according to claim 6.

8. The cosmetic according to claim 7, wherein the SPF value is 15 or more and the viscosity is 50000Pa·s or less.

9. The cosmetic according to claim 7 or 8, wherein the inorganic particle is contained in an amount of 5% by mass or more and 50% by mass or less based on the total amount of the cosmetic.
